# EUROPÄISCHE PATENTSCHRIFT

(11) **EP 1 531 890 B1**
(45) Veröffentlichungstag und Bekanntmachung des Hinweises auf die Patenterteilung: **10.10.2007**
(21) Anmeldenummer: 03787561.4
(22) Anmeldetag: 07.08.2003
(51) Int. Cl.: A61M 5/315

(54) **INJEKTIONSVORRICHTUNG MIT EINER KLICKGERÄUSCHPRODUZIERENDEN DOSIEREINHEIT**
INJECTION DEVICE PROVIDED WITH A DOSING UNIT PRODUCING A CLICK SOUND
DISPOSITIF D'INJECTION A UNITE DE DOSAGE PRODUISANT UN BRUIT DE CLIC

(30) Priorität: 14.08.2002 DE 10237258
(43) Veröffentlichungstag der Anmeldung: 25.05.2005
(73) Patentinhaber: TecPharma Licensing AG, 3401 Burgdorf (CH)
(72) Erfinder: GRAF, Roney, CH-3400 Burgdorf (CH)
(86) Internationale Anmeldenummer: PCT/CH2003/000532
(87) Internationale Veröffentlichungsnummer: WO 2004/016307

(56) Entgegenhaltungen:
- WO-A-01/72361
- WO-A-01/95959
- WO-A-02/24260
- WO-A-93/16740
- WO-A-02/092153
- US-A- 5 820 602
- US-A- 5 827 232

## Beschreibung

Die vorliegende Erfindung betrifft eine Injektionsvorrichtung gemäß dem Oberbegriff des Patentanspruchs 1.

Eine Injektionsvorrichtung gemäß dem Oberbegriff des Patentanspruchs 1 ist aus EP 0 581 924 B1 der Anmelderin oder WO93/16740 bekannt. Die bekannte Injektionsvorrichtung dient zum Injizieren jeweils wählbarer Flüssigkeitsmengen eines Produkts, das vorzugsweise einen medizinischen oder therapeutischen Wirkstoff enthält, aus einem mit einem Kolben versehenen Produktbehälter, insbesondere aus einer Ampulle. Die Vorrichtung umfasst eine manuell antreibbare röhrenartige Betätigungseinrichtung, die umfasst: einen Bedienknopf, der axial und drehbeweglich ist, ein drehfest mit dem Bedienknopf verbundenes Antriebselement, das Bewegungen des Bedienknopfes folgt, ein Abtriebsglied, das drehfest in Bezug auf ein Gehäuse der Vorrichtung ist, sowie ein Führungselement für das Abtriebsglied.

Bei der bekannten Vorrichtung ist ein Antriebselement, an dem das Abtriebsglied bewegbar gelagert ist, axial, d. h. in Vorschubrichtung des Kolbens, von einer Ruhelage aus um eine durch die Mechanik vordefinierte Strecke zu einer vorderen Endstellung verschiebbar. Durch Verdrehen des Bedienknopfes wird die auszuschüttende Produktdosis eingestellt.

Zwischen drehfesten und drehbaren Teilen der bekannten Injektionsvorrichtung ist ein Drehraster vorgesehen, das zwei scheibenartige Rastelemente umfasst, die einander gegenüberliegen und jeweils eine Mehrzahl von Rastvorsprüngen und von zu diesen vorzugsweise korrespondierenden Rastvertiefungen aufweisen, die miteinander zusammenwirken.

Beim Einstellen der zu injizierenden Dosis durch Verdrehen des Bedienknopfes gleiten die Rastvorsprünge an den Rastvertiefungen ab. Dabei wird ein Geräusch erzeugt, das in eindeutiger Weise von der Drehbewegung des Bedienknopfes abhängt. Bei der EP 0 581 924 B1 wird jeweils ein Klick-Geräusch erzeugt, wenn die Rastvorsprünge in die zu diesen korrespondierend ausgebildeten Rastvertiefungen zurückschnellen. Anhand des erzeugten Geräusches, insbesondere anhand der Anzahl von Klick-Geräuschen, kann der Patient durch Abzählen die Größe der neuen Injektionsdosis auch rein nach Gehör, d.h. auch ohne auf die Vorrichtung zu schauen, einstellen. Eine solche Einstellung der zu injizierenden Dosis nach Gehör hat sich als sehr vorteilhaft herausgestellt, insbesondere bei Patienten, bei denen es aufgrund einer Erkrankung zu einer Beeinträchtigung der Sehfähigkeit gekommen ist, wie dies beispielsweise bei Diabetes-Patienten häufig der Fall ist.

Beim Stand der Technik besteht das Drehraster aus zwei scheibenförmigen Rastelementen, auf denen in Umfangsrichtung in regelmäßigen Abständen sägezahnförmige Rastvorsprünge bzw. -vertiefungen angeordnet sind. Die beiden Rastelemente werden mittels einer als Rückstellelement dienenden Feder gegeneinandergedrückt, so dass sich das Drehraster bzw. der Bedienknopf nur in einer Drehrichtung verdrehen lässt, in der anderen Drehrichtung jedoch gesperrt ist. Dies hat den Nachteil, dass eine einmal durch Verdrehen des Bedienknopfes in die erste Drehrichtung eingestellte Dosis nur noch weiter erhöht werden kann, nämlich durch weiteres Verdrehen des Bedienknopfes in die erste Richtung, nicht jedoch durch Zurückdrehen des Bedienknopfes in die entgegengesetzte Drehrichtung wieder verkleinert werden kann. Hat der Patient also versehentlich eine zu große Dosis gewählt, etwa weil er die Anzahl von Klick-Geräuschen falsch gezählt hat, so muss die falsch gewählte Dosis durch Vorschieben des Abtriebsglieds in die vordere Endstellung zuerst ausgeschüttet werden und das Abtriebsglied wieder zurück in die Ruhelage gebracht werden, bevor in einem neuen Versuch eine neue Dosis eingestellt werden kann. Diese Vorgehensweise ist mühsam, fehlerträchtig und verschwendet unnötig viel Produkt.

Aufgabe der vorliegenden Erfindung ist es, die gattungsgemäße Injektionsvorrichtung dahingehend weiterzubilden, dass die zu verabreichende Dosis in einfacher Weise zuverlässig vorgebbar ist, wobei eine einmal gewählte Dosis jederzeit auch wieder reduzierbar sein soll.

Diese Aufgabe wird durch eine Injektionsvorrichtung mit den Merkmalen nach Patentanspruch 1 gelöst. Vorteilhafte Weiterbildungen sind Gegenstand der rückbezogenen Unteransprüche.

Eine erfindungsgemäße Injektionsvorrichtung zeichnet sich dadurch aus, dass das Drehraster so ausgelegt ist, dass der Bedienknopf in dessen Ruhelage, d.h. wenn das Abtriebsglied beabstandet zum Kolben ist, in beide Drehrichtungen bewegbar ist. Weil die erfindungsgemäße Vorrichtung auch weiterhin ein Drehraster aufweist, das beim Verdrehen akustische Geräusche, vorzugsweise Klick-Geräusche, abgibt, kann der Patient auch weiterhin die Dosis nach dem Gehör, insbesondere anhand der Anzahl von Klick-Geräuschen, vorgeben. Dies gewährleistet eine einfache und zuverlässige Dosierung. Weil erfindungsgemäß das Drehraster in der Ruhelage in beide Drehrichtungen bewegbar ist, kann auch ohne unnötige Produktausschüttung, wie sie vorstehend erläutert wurde, die einmal gewählte Dosis in einfacher Weise wieder reduziert werden.

Vorzugsweise erzeugt das Drehraster nicht nur beim Verdrehen des Bedienknopfes in die erste Drehrichtung, beispielsweise im Uhrzeigersinn, das vorgenannte akustische Geräusch, sondern auch beim Verdrehen des Bedienknopfes in die entgegengesetzte Drehrichtung, d.h. beispielsweise im Gegenuhrzeigersinn. Somit kann der Patient sich auch beim Zurückdrehen des Bedienknopfes bzw. des Drehrasters anhand des erzeugten akustischen Geräusches orientieren und auf die jeweils eingestellte Dosis rückschließen. Die erfindungsgemäße Injektionsvorrichtung kann deshalb in einfacher Weise auch ohne Ablesen derselben zuverlässig bedient werden.

Gemäß einer bevorzugten Ausführungsform ist der Bedienknopf und gemeinsam mit diesem das Abtriebsglied nur bei eingerastetem Drehraster, wenn die Rastvorsprünge im Wesentlichen vollständig mit den zu diesen korrespondierend ausgebildeten Rastvertiefungen zusammenwirken, axial aus der Ruhelage in die vordere Endlage verstellbar. Auf diese einfache Weise kann gewährleistet werden, dass nur eine Dosis verabreicht werden kann, die durch Verdrehen des Bedienknopfes um ein ganzzahliges Vielfaches der Umfangswinkelabstände der Rastvorsprünge eingestellt wird.

Bei der vorgenannten Ausführungsform sind bevorzugt am Außenumfang des Bedienknopfes oder an einem zu diesem drehfest angeordneten Element, beispielsweise an dem Gehäuse der Injektionsvorrichtung, eine Anzahl von Rastkörpern, beispielsweise in Axialrichtung verlaufende Vertiefungen und korrespondierend hierzu ausgebildete in Axialrichtung verlaufende Vorsprünge, ausgebildet, wobei die Anzahl der Rastkörper bevorzugt der Anzahl von Rastvorsprüngen entspricht, die auf den Stirnseiten der Rastelemente des Drehrasters ausgebildet sind. Nur wenn die Rastkörper ein axiales Vorschieben des Bedienknopfes und des an diesen gekoppelten Abtriebsglieds zulassen, kann die eingestellte Dosis auch tatsächlich verabreicht werden.

Bevorzugt erzeugen auch die vorgenannten Rastkörper ein akustisches Geräusch, insbesondere Klick-Geräusche, die ganz besonders bevorzugt synchronisiert zu den akustischen Geräuschen, insbesondere Klick-Geräuschen, des Drehrasters erzeugt werden, so dass die Winkelpositionen der Rastkörper bevorzugt im wesentlichen den Winkelpositionen der Rastvorsprünge bzw. Rastvertiefungen des Drehrasters entsprechen.

Gemäß einer bevorzugten Ausführungsform ist ein zweites Rückstellelement vorgesehen, das einen Ampullenhalter, der ein erstes Rastelement, beispielsweise eine untere Scheibe des Drehrasters, ausbildet oder mit diesem gekoppelt ist, axial in Richtung des Bedienknopfes zurückschiebt, so dass das Drehraster einrastet, wobei in der eingerasteten Position des Drehrasters die Rastvorsprünge im Wesentlichen vollständig mit den zu diesen korrespondierend ausgebildeten Rastvertiefungen zusammenwirken, beispielsweise in diese nahezu vollständig eingreifen. Das zweite Rückstellmittel kann beispielsweise zwischen dem hinteren Teil der Injektionsvorrichtung, wo die manuell antreibbare Betätigungseinrichtung sitzt, und dem Ampullenhalter sitzen.

Ganz besonders bevorzugt ist die Rückstellkraft des zweiten Rückstellelements kleiner als diejenige des ersten Rückstellelements, so dass das Drehraster nicht starr, sondern vielmehr gefedert gelagert ist, so dass die von den Rückstellelementen in gegenseitige Anlage gebrachten Rastelemente des Drehrasters dann, wenn die Rastvorsprünge an den Rastvertiefungen abgleiten, federnd zurückschnellen, was das erzeugte akustische Geräusch noch besser hörbar macht.

Die vorliegende Erfindung ist grundsätzlich auf die in EP 0 581 924 B1 beschriebene Vorrichtung anwendbar, wenngleich die vorliegende Erfindung grundsätzlich nicht auf die dort beschriebene spezielle Ausführungsform beschränkt ist. Bei einer solchen Ausführungsform ist ein Antriebselement, an dem das Abtriebsglied bewegbar gelagert ist, axial, d.h. in der Vorschubrichtung des Kolbens, von einer Ruhelage aus um eine durch die Mechanik vordefinierte Strecke zu einer vorderen Endstellung verschiebbar. Über einen Teil der vordefinierten Strecke ist das Abtriebsglied nicht in Anlage zu dem Kolben des Produktbehälters. Über den restlichen Teil der vordefinierten Strecke ist das Abtriebsglied in Anlage zu dem Kolbenstopfen, so dass der Kolben beim weiteren Vorschieben des Abtriebsglieds um den restlichen Teil der vordefinierten Strecke axial vorgeschoben wird, so dass ein Produkt, bevorzugt eine Flüssigkeit, aus dem Produktbehälter ausgeschüttet wird. Nach der Ausschüttung des Produkts wird das Abtriebsglied mit Hilfe eines Rückstellmittels in die Ruhelage zurückgestellt. Beim erneuten axialen Vorschieben des Abtriebsglieds um die für die vorherige Injektion eingestellte Wegstrecke würde, weil das Abtriebsglied nicht in Anlage zu dem Kolben gelangen kann, kein weiteres Produkt aus dem Produktbehälter ausgeschüttet werden.

Die Dosierung geschieht bei dieser Ausführungsform wie folgt: In der Ruhelage ist der Bedienknopf drehbeweglich. Beim Verdrehen des Bedienknopfes in der Ruhelage, beispielsweise im Uhrzeigersinn, wird das sich dann nicht in Anlage zu dem Kolben befindliche Abtriebsglied kontrolliert axial vorgeschoben, was den Abstand zwischen dem Abtriebsglied und dem Kolben verkürzt. Die Strecke, um die der Abstand zwischen dem Abtriebsglied und dem Kolben beim Verdrehen des Bedienknopfes verkürzt wird, entspricht der Wegstrecke, während der das Abtriebsglied beim Vorschieben in die vordere Endlage in Anlage zu dem Kolben gelangt, um eine weitere Produktdosis aus dem Produktbehälter auszuschütten. Der Drehwinkel, um den der Bedienknopf in die erste Richtung, beispielsweise im Uhrzeigersinn, gedreht wird, legt somit die auszuschüttende Produktdosis eindeutig fest.

Nachfolgend werden bevorzugte Ausführungsbeispiele anhand der beigefügten Zeichnungen beschrieben werden, worin:
- Figur 1: einen Längsschnitt durch eine Injektionsvorrichtung nach dem Stand der Technik darstellt;
- Figur 2: in einem Längsschnitt schematisch ein Drehraster gemäß einer ersten Ausführungsform der vorliegenden Erfindung darstellt;
- Figur 3: in einer Abwicklung schematisch eine zweite Ausführungsform eines erfindungsgemäßen Drehrasters darstellt;
- Figur 4: den Querschnitt A-A der Injektionsvorrichtung gemäß der Figur 1 darstellt;
- Figur 5: den Querschnitt B-B der Injektionsvorrichtung gemäß der Figur 1 darstellt;
- Figur 6: den Querschnitt C-C der Injektionsvorrichtung gemäß der Figur 1 darstellt;
- Figur 7: in einer vergrößerten Darstellung die Rastvorsprünge der Figur 3 darstellt; und
- Figur 8: die Abwicklung gemäß der Figur 3 in drei verschiedenen Winkelstellungen des Bedienknopfes der erfindungsgemäßen Injektionsvorrichtung darstellt.

In den Figuren bezeichnen identische Bezugszeichen identische oder im Wesentlichen gleichwirkende Elemente und Funktionsgruppen.

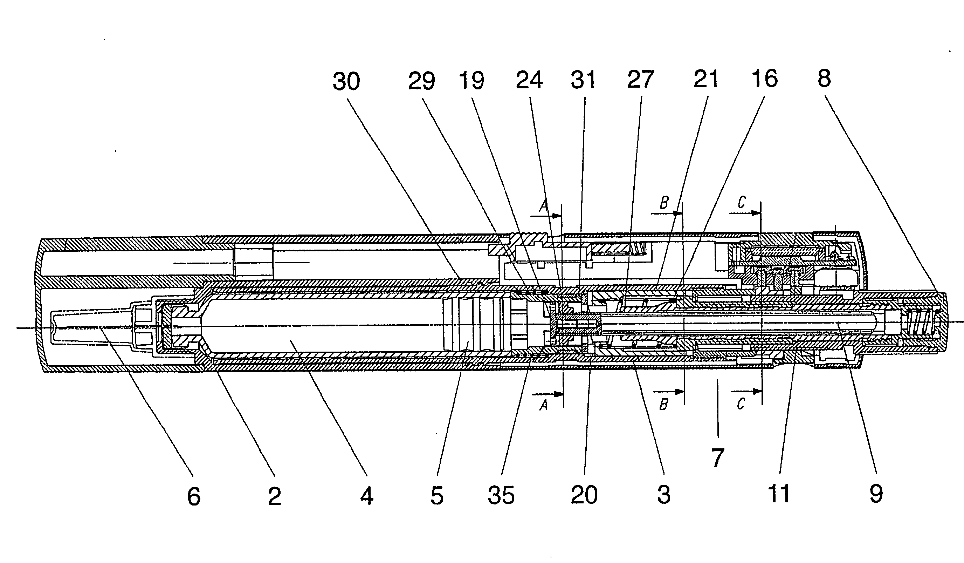

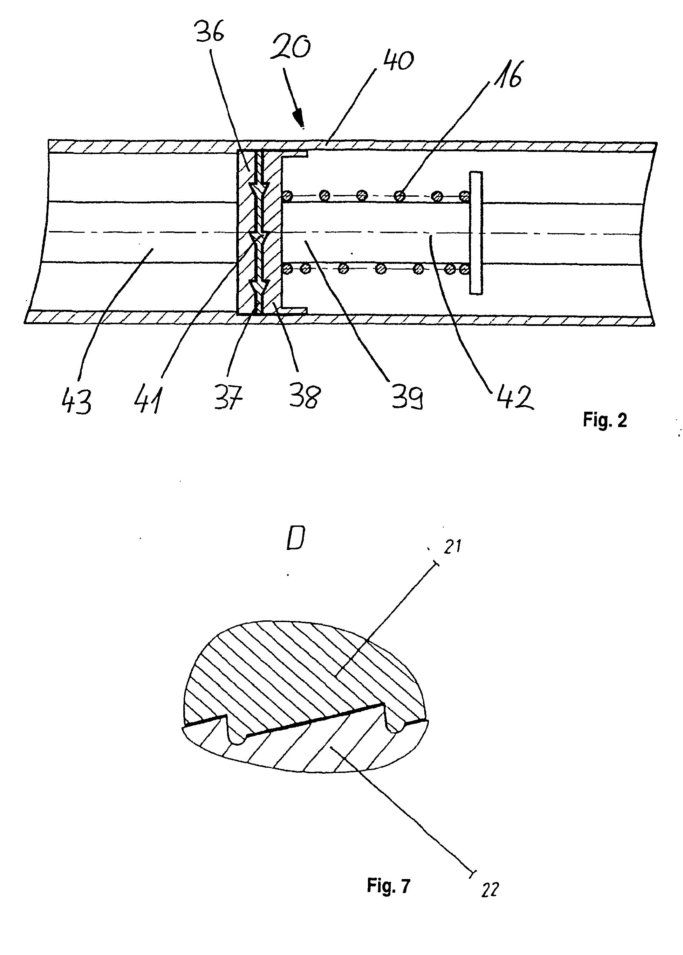

Um die Funktionsweise einer erfindungsgemäßen Injektionsvorrichtung besser zu verdeutlichen, wird nachfolgend zunächst die in der Figur 1 in einem Längsschnitt gezeigte gattungsgemäße Injektionsvorrichtung beschrieben werden, die aus der Figur 2 der EP 0 581 924 B1 der Anmelderin bekannt ist. Die vorliegende Erfindung ist jedoch nicht auf diese spezielle Ausführungsform beschränkt. Die Injektionsvorrichtung umfasst in einem vorderen Teil 2 eine austauschbare Ampulle 4, in der ein Kolben 5 axial verstellbar sitzt, der die in der Ampulle aufbewahrte Substanz, die bevorzugt einen medizinischen oder therapeutischen Wirkstoff enthält, durch eine Injektionsnadel 6 ausschüttet. In dem hinteren Teil 3 besitzt die Vorrichtung eine von Hand bedienbare röhrenförmig aufgebaute Betätigungseinrichtung 7. Diese umfasst einen Bedienknopf 8, ein im Wesentlichen als Stab 9 mit einem Flansch 19 ausgebildetes Abtriebsglied, ein Führungselement 24 und ein Antriebselement 11.

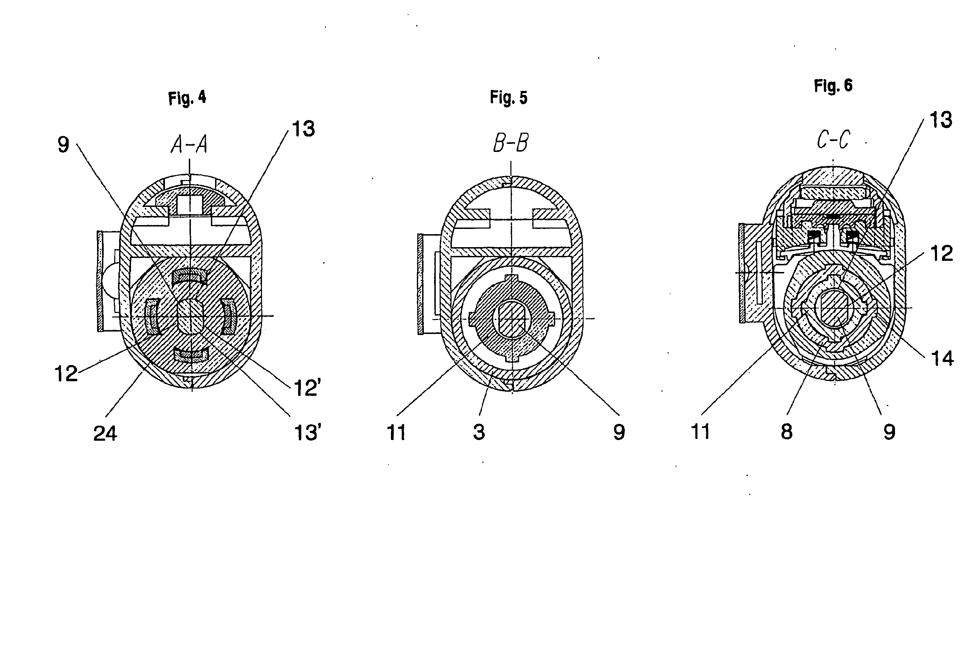

Wie in den Querschnitten gemäß den Figuren 4 bis 6 dargestellt, besitzt der Stab 9 an beiden Seiten plane Flächen und weist im übrigen einen kreisförmigen Querschnitt auf, der ein Gewinde trägt. Dieses Gewinde sitzt in einem Muttergewinde 27 des Antriebselements 11, das drehfest in der Injektionsvorrichtung sitzt. Das Antriebselement 11 ist zusammen mit der gesamten Betätigungseinrichtung 7 durch axiales Betätigen des Bedienknöpfes 8 und Vermittlung dieser Bewegung über das Führungselement 24 gegen die Kraft einer als erstes Rückstellmittel dienenden Feder 16 von einer (hinteren) Ruhelage in eine (vordere) Endlage überführbar. Der Stab 9 macht diese axiale Vorschubbewegung mit. Dabei drückt schließlich der am Stab 9 befindliche Flansch 19 gegen den Kolben 5 der Ampulle 4, schiebt diesen nach vorne und bewirkt so die Injektion.

Der Stab 9 sitzt drehfest aber axial beweglich im Führungselement 24, das seinerseits drehfest mit dem Bedienknopf 8 verbunden ist. Wie in dem Querschnitt gemäß der Figur 4 dargestellt ist, besitzt das Führungselement 24 eine Innenkontur, die (bis auf etwaige Toleranzen) an die Außenkontur des Stabs 9, wie sie vorstehend beschrieben wurde, angepasst ist. Der Bedienknopf 8, das Führungselement 24 und der Stab 9 lassen sich nur in der (hinteren) Ruhelage der Betätigungseinrichtung 7 drehen. Dabei dreht sich der Stab 9 im Muttergewinde 27 des in der Injektionsvorrichtung drehfest angeordneten Antriebselements 11 und verstellt sich so nach vorne, wenn der Betätigungsknopf 8 in eine erste Drehrichtung, beispielsweise im Uhrzeigersinn, gedreht wird. Wie nachfolgend noch dargelegt werden wird, kann der Bedienknopf in dem in der Figur 1 gezeigten Zustand gemäß dem Stand der Technik nicht in die entgegengesetzte Drehrichtung, d.h. im vorgenannten Fall entgegen dem Uhrzeigersinn, zurückgedreht werden.

Wie zuvor beschrieben wurde, wird die Injektion der Substanz durch axiales Vorschieben des Bedienknopfes 8 und des als Abtriebsglied dienenden Flansches 19 hin zu der vorderen Endlage bewirkt. Der Bedienknopf und damit der Flansch 19 lässt sich dabei jeweils nur um eine einheitliche, vordefinierte Strecke vorschieben, die beispielsweise durch Anschläge in der Betätigungseinrichtung 7 und durch den maximalen Hub des Bedienknopfes 8 vorgegeben ist. Wird der Bedienknopf 8 nach einer Injektion in die (hintere) Ruhelage zurückgeführt, so würde bei einem erneuten axialen Vorschieben des Bedienknopfes 8 und des Flansches 19 keine weitere Substanz aus der Ampulle 4 ausgeschüttet werden. Zur Ausschüttung einer neuen Dosis muss der Flansch 19 durch Verdrehen des Bedienknopfes 8 in die erste Drehrichtung um eine der zu verabreichenden Dosis entsprechende Strecke vorgeschoben werden, so dass sich der Abstand zwischen dem als Abtriebsglied dienenden Flansch 19 und dem Kolben 5 um diese vorgebbare Strecke verkürzt. Somit gelangt beim erneuten Vorschieben des Bedienknopfes 8 und des als Abtriebsglied dienenden Flansches 19 der Flansch 19 zum Ende seiner axialen Vorschubbewegung in Anlage zu dem Kolben 5 und verschiebt diesen um die durch die Verdrehbewegung des Bedienknopfes 8 eingestellte Strecke axial nach vorne, so dass eine neue Dosis aus der Ampulle 4 ausgeschüttet wird, die eindeutig durch die Verdrehung des Bedienknopfes 8 festgelegt ist.

Das röhrenförmige Antriebselement 11 ist mit dem Bedienknopf 8 fest gegen Verdrehung verbunden. Der axial bewegliche Bedienknopf 8 ist nur in seiner (hinteren) Ruhelage drehbeweglich, und zwar gemäß EP 0 581 924 B1 nur in einer Drehrichtung, um ein axiales Vorschieben des als Abtriebsglied dienenden Flansches 19 zu bewirken. Im Inneren des Antriebselements 11 ist der Stab 9 gehalten, dessen Gewindeteile an den Kreisflächen 13, 13' in das Muttergewinde 27 des Antriebselements 11 eingreifen. Der Stab 9 durchgreift das Antriebselement 11 und das Führungselement 24. Letzteres ist fest mit dem hinteren Teil 3 der Injektionsvorrichtung verbunden und kann weder eine axiale Bewegung noch eine Drehbewegung ausführen. Die an den Stab 9 angepasste Öffnung in dem Führungselement 24 bewirkt, dass der Stab 9 nur axiale Bewegungen, aber keine Drehbewegungen ausführen kann.

Wird der Bedienknopf 8 manuell axial vorgeschoben, so verschiebt er das Antriebselement 11 bis zu seiner (vorderen) Endlage, die durch einen Anschlag 31, beispielsweise des Antriebselements 11 am Führungselement 24, festgelegt ist. Diese axiale Bewegung wird auf den im Muttergewinde 27 des Antriebselements 11 sitzenden Stab 9 übertragen, der nur axiale Bewegungen, aber keine Drehbewegungen ausführen kann.

Die axiale Bewegung wird gegen die Kraft einer als erstes Rückstellmittel dienenden Feder 16 ausgeführt, die in einer Aussparung zwischen dem die axiale Bewegung ausführenden Antriebselement 11 und einem Hülsenteil 21 des nachfolgend zu beschreibenden Drehrasters 20 sitzt. Die Feder 16 bringt die Betätigungseinrichtung 7 wieder in die Ruhelage zurück.

Weil der Stab 9 in dem Führungselement 24 drehfest gelagert ist, kann die zur Einstellung der nächsten Injektionsdosis am Bedienknopf 8 ausgeübte Drehbewegung nicht auf den Stab 9 übertragen werden. Vielmehr wird durch das sich drehende Muttergewinde 27 des Führungselements 11 der Stab 9 über die Gewindeteile an den Kreisflächen 13, 13' (vergleiche Figur 4) drehfest axial nach vorne getrieben und so der Flansch 19 in die Stellung gebracht, die der nächsten abzugebenden Injektionsdosis entspricht. Beim axialen Vorschieben des Flansches 19 verringert sich der Abstand zwischen dem Flansch 19 und dem Kolben 5 entsprechend der Verdrehung des Bedienknopfes 8.

Der Weg des Flansches 19 von der (hinteren) Ruhelage in die (vordere) Endlage der Betätigungseinrichtung 7 bleibt stets gleich und entspricht der konstanten Distanz, um die der Flansch 19 vor Einstellung der Injektionsdosis vom Kolben 5 getrennt ist. Durch Verdrehen des Bedienknopfes 8 in der (hinteren) Ruhelage kann somit die zu verabreichende Injektionsdosis im Voraus, d.h. vor der Produktausschüttung aus der Ampulle 4, eingestellt werden. Durch kontrolliertes Verdrehen des Bedienknopfes 8 in der ersten Drehrichtung kann somit die Injektionsdosis eindeutig festgelegt werden.

Damit die Injektionsdosis auch nach Gehör anhand akustischer Geräusche zuverlässig eingestellt werden kann, ist aus der gattungsbildenden EP 0 581 924 B1 ein zweiteiliges Drehraster bekannt, das ein Verdrehen des Bedienknopfes 8 nur in die erste Drehrichtung, beispielsweise im Uhrzeigersinn, zulässt und die Verdrehung in die entgegengesetzte zweite Drehrichtung, beispielsweise im Gegenuhrzeigersinn, sperrt. Beim Verdrehen des Drehrasters gleiten Rastvorsprünge an Rastvertiefungen ab, so dass ein definiertes akustisches Geräusch, nämlich ein Klick-Geräusch, in eindeutiger Zuordnung zu der Drehbewegung des Bedienknopfes 8 erzeugt wird.

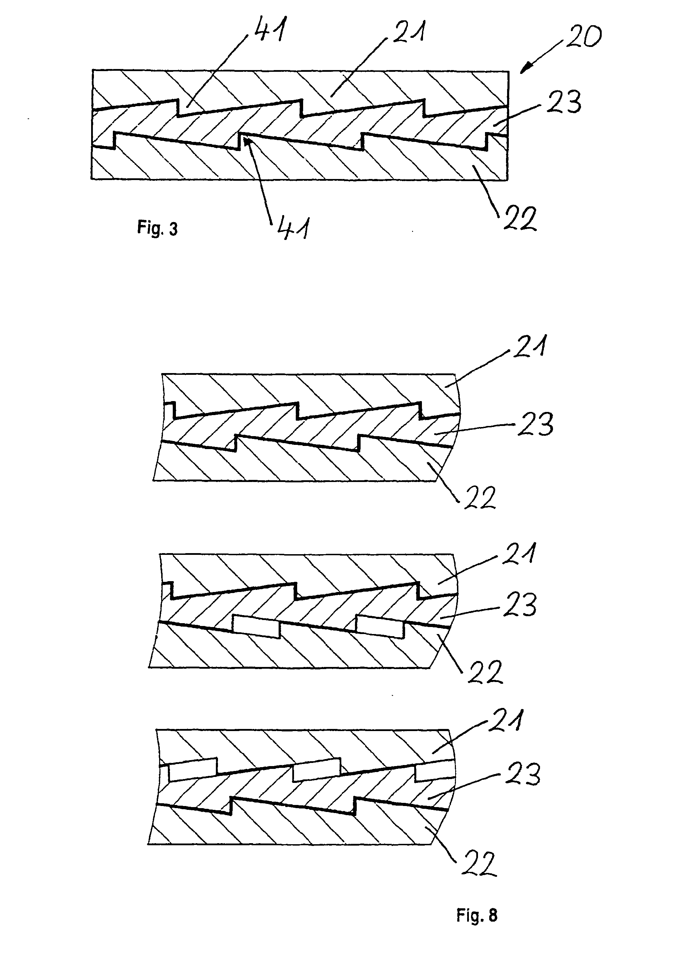

Das Drehraster 20 sitzt allgemein zwischen den drehfesten und den drehbaren Teilen der Injektionsvorrichtung. Das Drehraster 20 ist schematisch in einer Abwicklung in der Figur 7 dargestellt. Gemäß EP 0 581 924 B1 umfasst das Drehraster 20 zwei in der vorgenannten Ruhelage aneinander anliegende Rastelemente 21, 22, die eine Mehrzahl von in Umfangsrichtung verlaufenden und vorzugsweise in regelmäßigen Winkelabständen angeordneten sägezahnförmigen Rastvorsprüngen bzw. korrespondierend zu diesen ausgebildeten Rastvertiefungen aufweisen, wie in der Figur 7 dargestellt. Das Rastelement 22 entspricht dem Ampullenhalter 29 oder ist mechanisch an diesen gekoppelt. Das Rastelement 21 entspricht einem drehfest mit dem Bedienknopf 8 und drehbeweglich gelagerten Hülsenteil des röhrenförmigen Antriebselements 11.

Beim Verdrehen der beiden Rastelemente 21, 22 relativ zueinander gleiten die sägezahnförmigen Rastvorsprünge an den korrespondierend hierzu ausgebildeten Rastvertiefungen ab. Weil die beiden Rastelemente 21, 22 mittels der Rückstellkraft der als erstes Rückstellmittel dienenden Feder 16 gegeneinander gedrückt werden, schnellen die beiden Rastelemente 21, 22 nach vollständigem Abgleiten eines sägezahnförmigen Rastvorsprungs zurück, was ein deutlich hörbares Klick-Geräusch erzeugt, das als akustisches Geräusch zum Einstellen der Injektionsdosis "nach Gehör" dient. Das Drehraster gemäß EP 0 581 924 B1 sowie entsprechend nach der WO 87/02895 ist nur in einer Drehrichtung drehbeweglich. Wird also der Bedienknopf 8 zu weit in die erste Drehrichtung gedreht, etwa weil ein Benutzer die Anzahl von Klick-Geräuschen falsch gezählt hat, so kann der Bedienknopf 8 nicht zurückgedreht werden. Die falsch eingestellte Injektionsdosis kann deshalb nicht wieder verkleinert werden. Vielmehr muss Substanz durch axiales Vorschieben des Bedienknopfes 8 unnötigerweise ausgeschüttet werden und kann eine neue Injektionsdosis erst nach Rückkehr des Bedienknopfes 8 in die (hintere) Ruhelage neu eingestellt werden.

Gemäß der vorliegenden Erfindung umfasst das Drehraster 20 mehr als zwei relativ zueinander verdrehbare Rastelemente, bevorzugt genau drei relativ zueinander verdrehbare Rastelemente. Die Figuren 2 und 3 zeigen schematisch zwei Ausführungsbeispiele der vorliegenden Erfindung.

Die Figur 2 zeigt in einem Teil-Längsschnitt ein erstes Ausführungsbeispiel, bei dem das Drehraster 20 eine untere Schiebehülse 36, einen als Zwischenscheibe dienenden Pfeilring 37 mit Rastvorsprüngen 41 und eine obere Schiebehülse 38 umfasst. Die obere Schiebehülse 38 ist drehfest mit dem nicht dargestellten Bedienknopf 8 verbunden, beispielsweise über das schematisch dargestellte zylindrische Zwischenstück 42. Die untere Schiebehülse 36 ist bevorzugt mit dem Ampullenhalter 29 verbunden, beispielsweise über das schematisch dargestellte zylindrische Zwischenstück 43.

Die als Rastelemente dienenden scheibenförmigen Schiebehülsen 36, 38 und der Pfeilring 37 weisen eine Mehrzahl von Rastvorsprüngen 41 und korrespondierend zu diesen ausgebildeten Rastvertiefungen auf.

Wird die obere Schiebehülse 38 nach links verdreht, so gleiten die in dieser ausgebildeten Rastvertiefungen an den Rastvorsprüngen 41 ab, was zu einem axialen Rückschieben gegen die Rückstellkraft der Feder 16 führt. Beim weiteren Verdrehen der oberen Schiebehülse 38 nach links greift schließlich die Rastvertiefung in den nächsten Rastvorsprung 41 ein. Aufgrund der Rückstellkraft der Feder 16 schnellt die obere Schiebehülse 38 zurück und erzeugt dabei ein hörbares Klick-Geräusch. Aufgrund der gleichgerichteten PF-Teilung der Rastvorsprünge 41 auf dem Pfeilring 37 ist dieser beim Verdrehen der oberen Schiebehülse 38 nach links gesperrt, bleibt also in den Rastvertiefungen, die auf der Stirnseite der oberen Schiebehülse 36 ausgebildet sind.

Wird der nicht dargestellte Bedienknopf 8 hingegen in der Figur 2 nach rechts verdreht, also in die entgegengesetzte Drehrichtung, so bleiben die Rastvorsprünge 41 auf der oberen Stirnseite des Pfeilrings 37 und die auf der Stirnseite der oberen Schiebehülse 38 ausgebildeten Rastvertiefungen ineinander verhakt und gleiten die auf der unteren Stirnseite des Pfeilrings 37 ausgebildeten Rastvertiefungen an den auf der oberen Stirnseite der unteren Schiebehülse 36 ausgebildeten Rastvertiefungen ab. Dabei werden Pfeilring 37 und obere Schiebehülse 38 axial gegen die Rückstellkraft der Feder 16 zurückgeschoben.

Beim weiteren Verdrehen des Bedienknopfes 8 nach rechts greifen schließlich die auf der unteren Stirnseite des Pfeilrings 37 ausgebildeten Rastvorsprünge in die nächste Rastvertiefung auf der oberen Stirnseite der unteren Schiebehülse 36 ein. Dabei schnellen obere Schiebehülsen 38 und Pfeilring 37 aufgrund der Rückstellkraft der Feder 16 zurück, was ein deutlich wahrnehmbares Klick-Geräusch erzeugt.

Erfindungsgemäß werden deshalb Klick-Geräusche oder vergleichbare akustische Geräusche beim Verdrehen des Bedienknopfes 8 sowohl im Uhrzeigersinn als auch im Gegenuhrzeigersinn erzeugt. Der Patient muss sich also nur merken, in welche Richtung er den Bedienknopf 8 dreht, und kann so aus der Anzahl von Klickgeräuschen eindeutig auf die eingestellte Injektionsdosis rückschließen. Hat er versehentlich durch Drehen des Bedienknopfes 8 in die erste Drehrichtung, beispielsweise im Uhrzeigersinn, eine zu große Injektionsdosis eingestellt, so kann er den Bedienknopf 8 erfindungsgemäß wieder in die entgegengesetzte Drehrichtung, d.h. beispielsweise im Gegenuhrzeigersinn, zurückdrehen und die Injektionsdosis wieder verkleinern. Auch beim Zurückdrehen werden Klick-Geräusche oder vergleichbare akustische Geräusche erzeugt, aus denen der Benutzer auf die Injektionsdosis rückschließen kann.

In dem Ausführungsbeispiel gemäß der Figur 2 sind die auf einander gegenüberliegenden Stirnseiten des als Zwischenscheibe dienenden Pfeilrings 37 vorgesehenen Rastvorsprünge 41 an, in Vorschubrichtung des Kolbens 5 betrachtet, zueinander fluchtenden Winkelpositionen angeordnet.

Die Figur 3 zeigt ein weiteres Ausführungsbeispiel gemäß der vorliegenden Erfindung, bei der Rastvorsprünge auf einer jeweils als Rastelement dienenden oberen und unteren Scheibe in der Ruhestellung des Bedienknopfes 8, in Vorschubrichtung des Kolbens 5 betrachtet, an relativ zueinander versetzten Winkelpositionen angeordnet sind. Gemäß der Figur 3 umfasst das Drehraster 20 eine obere Scheibe 21, eine untere Scheibe 22 und eine Zwischenscheibe 23. Die obere Scheibe 21 und die untere Scheibe 22 weisen eine Mehrzahl von vorzugsweise in regelmäßigen Winkelabständen angeordneten sägezahnförmig verlaufenden Rastvorsprüngen 41 auf. Korrespondierend zu diesen weist auch die Zwischenscheibe 23 Rastvorsprünge und Rastvertiefungen auf. Die obere Scheibe 21 wird durch die Rückstellkraft einer als erstes Rückstellmittel dienenden Feder 16 (nicht dargestellt) zu der unteren Scheibe 22 gedrückt. In der Ruhelage des Bedienknopfes 8 greifen die Rastvorsprünge 41 bevorzugt im Wesentlichen vollständig in die korrespondierend zu diesen ausgebildeten Rastvertiefungen ein.

Beim Verdrehen der oberen Scheibe 21 relativ zu der unteren Scheibe 22 ergibt sich der schematisch in den Abwicklungen gemäß der Figur 8 dargestellte Bewegungsablauf. Die Figur 8A zeigt den Ausgangszustand des Drehrasters 20 in der Ruhelage des nicht dargestellten Bedienknopfes 8, wo die Gesamthöhe des Drehrasters 20 minimal ist. Beim Verdrehen der oberen Scheibe 21, die drehfest mit dem nicht dargestellten Bedienknopf 8 verbunden ist, nach links bleiben die auf der oberen Stirnseite der Zwischenscheibe 23 ausgebildeten Rastvorsprünge in den auf der unteren Stirnseite der oberen Scheibe 21 ausgebildeten Rastvertiefungen ineinander verhakt, so dass die auf der unteren Stirnseite der Zwischenscheibe 23 ausgebildeten Rastvorsprünge an den korrespondierend zu diesen ausgebildeten Rastvertiefungen auf der oberen Stirnseite der unteren Scheibe 22 abgleiten. Beim weiteren Verdrehen erreichen schließlich die auf der unteren Stirnseite der Zwischenscheibe 23 ausgebildeten Rastvorsprünge die nächsten Rastvertiefungen. Aufgrund einer Rückstellkraft werden dann die Scheiben 21, 22, 23 aneinander gedrückt, so dass die in der Figur 8A dargestellte Ausgangslage des Drehrasters 20 wieder eingenommen wird. Beim Zurückschnellen der oberen Scheibe 21 und der Zwischenscheibe 23 wird ein wahrnehmbares Klick-Geräusch erzeugt.

Beim Verdrehen der oberen Scheibe 21 in die entgegengesetzte Drehrichtung, d.h. in der Figur 8c nach rechts, ergibt sich ein entsprechender Bewegungsablauf, der sich der Figur 8c ohne Weiteres entnehmen lässt.

Die diversen Rastelemente des Drehrasters 20, d.h. die Scheiben 36 bis 38 in dem Ausführungsbeispiel gemäß der Figur 2 bzw. die Scheiben 21 bis 23 des zweiten Ausführungsbeispiels gemäß der Figur 3, werden wie folgt mit Hilfe von zwei Rückstellmitteln, bevorzugt Federn, zurückgestellt und in gegenseitige Anlage zueinander gebracht: Wie in der Figur 1 dargestellt, ist der vordere Teil 2 über das Grobgewinde 30 mit dem hinteren Teil 3 der Injektionsvorrichtung verbunden. Während das röhrenförmige Antriebselement 11 in dem hinteren Teil 30 sitzt, sitzt die Ampulle 4 in dem vorderen Teil 2 der Injektionsvorrichtung. Ein Ampullenhalter 29, der bevorzugt hohlzylindrisch ausgebildet ist und von dem als Abtriebsglied dienenden Flansch 19 durchgriffen wird, liegt auf dem oberen Stirnrand der Ampulle 4 auf. Zur federnden Lagerung der Ampulle 4 in dem vorderen Teil 2 der Injektionsvorrichtung sitzt zwischen dem Ampullenhalter 29 und dem hinteren Teil 3 eine als zweites Rückstellmittel dienende Feder 35. Sitzt die Ampulle 4 locker auf dem Ampullenhalter 29 auf, etwa weil das vordere Teil 2 noch nicht vollständig auf das Grobgewinde 30 aufgeschraubt ist, so schiebt die zweite Feder 35 die Ampulle 4 und den Ampullenhalter 29 axial nach vorne. Weil die eine Seite des erfindungsgemäßen Drehrasters 20, beispielsweise in der Figur 2 die untere Scheibe 36 oder in der Figur 3 die untere Scheibe 22, mit dem Ampullenhalter 29 gekoppelt ist oder von diesem gebildet wird, gelangt so das Drehraster 20 außer Eingriff. In dieser Stellung kann der Bedienknopf 8 ohne Rastwiderstände zurückgedreht werden, beispielsweise zum vollständigen Zurückfahren des Flansches 19 und des Stabs 9 in eine Ausgangslage, wenn eine neue Ampulle 4 eingesetzt werden soll, deren Kolben 5 anfangs in der hintersten Endstellung ist.

Die Länge der Ampulle 4 und die Länge einer zur Aufbewahrung der Ampulle 4 in dem vorderen Teil 2 dienenden Aufnahme ist so aufeinander abgestimmt, dass das Drehraster dann, wenn das vordere Teil 2 vollständig auf das Grobgewinde 30 aufgeschraubt wird, das erfindungsgemäße Drehraster vollständig einrastet, also eine Ausgangsstellung einnimmt, in der die Rastvorsprünge und korrespondierend zu diesen ausgebildete Rastvertiefungen im Wesentlichen vollständig und ganzflächig ineinander eingreifen, wie dies beispielsweise in der Figur 2 oder in den Abwicklungen gemäß der Figur 3 und 8a dargestellt ist.

Bevorzugt ist die zweite Feder 35 in dieser Ausgangsstellung nicht vollständig komprimiert, so dass beim Verdrehen des erfindungsgemäßen Drehrasters 20 die vorstehend anhand den Figuren 2, 3 und 8 beschriebenen Bewegungsabläufe der Rastelemente des Drehrasters 20 ausgeführt werden können, um akustische Geräusche, bevorzugt Klick-Geräusche, zu erzeugen.

Bevorzugt kann der Bedienknopf 8 zur Ausschüttung der eingestellten Dosis nur dann axial von der (hinteren) Ruhelage in die (vordere) Endlage verschoben werden, wenn das Drehraster 20 eingerastet ist, also die in der Figur 2 bzw. in den Abwicklungen gemäß den Figuren 3 und 8a dargestellte Ausgangslage einnimmt, wo die Rastvorsprünge und die korrespondierend zu diesen ausgebildeten Rastvertiefungen im Wesentlichen vollständig ganzflächig ineinander eingreifen. Diese Maßnahme stellt sicher, dass die verabreichbaren Dosierungen nicht stufenlos eingestellt werden können, sondern nur in ganzzahligen Vielfachen von kleinsten Dosen, die durch den Winkelabstand der Rastvorsprünge des Drehrasters 20 eindeutig vorgegeben sind. Gleichzeitig wird sichergestellt, dass beim Verdrehen des Drehrasters 20 um einen Rastvorsprung, entsprechend einer Erhöhung oder Verkleinerung der eingestellten Dosis um eine kleinste Dosis, ein Klick-Geräusch oder ein vergleichbares akustisches Geräusch erzeugt wird. Denn gemäß dieser bevorzugten Ausführungsform kann der Bedienknopf 8 nur dann axial verschoben werden, wenn das Drehraster 20 nach dem vorgenannten Zurückschnellen und dem dadurch erzeugten Klick-Geräusch wieder die in den Figuren 2, 3 bzw. 8a dargestellte Ausgangslage einnimmt.

Zu diesem Zweck können am Außenumfang des Bedienknopfes 8 oder an einem drehfest zu diesem angeordneten Element, beispielsweise einer Führungshülse, eine Anzahl von Rastkörpern angeordnet sein, wobei die Anzahl von Rastkörpern der Anzahl von Rastvorsprüngen auf den Stirnseiten der Rastelemente 21-23 bzw. 36-38 des erfindungsgemäßen Drehrasters 20 entspricht. Wie in der Figur 6, die einen Querschnitt entlang C-C gemäß der Figur 1 zeigt, schematisch dargestellt ist, trägt der Bedienknopf 8 in diesem Beispiel auf seinem Außenumfang vier unter gleichmäßigen Winkelabständen, d.h. unter 90°, angeordnete Rastkörper 14, die als axial verlaufende Vorsprünge ausgebildet sind. Der Figur 6 liegt dabei die nicht beschränkende Annahme zugrunde, dass das Drehraster auf den Stirnseiten der Rastelemente jeweils vier Rastvorsprünge bzw. Rastvertiefungen aufweist. Die Rastkörper 14 können in ihrer Winkelstellung zu den Rastvorsprüngen bzw. Rastvertiefungen auf den jeweiligen Stirnseiten des erfindungsgemäßen Drehrasters 20 fluchtend oder versetzt dazu angeordnet sein. Nur wenn eine korrespondierend zu dem Rastkörper 14 in dem den Bedienknopf 8 umgebenden Ring ausgebildete Axialnut fluchtet, kann der Bedienknopf 8 axial verschoben werden.

Bevorzugt ist diese Winkelstellung mit derjenigen Winkelstellung koordiniert, wo das Drehraster 20 eingerastet ist.

Bei der in der Figur 1 dargestellten Injektionsvorrichtung liegt das ortsfeste Führungselement 24 vor dem röhrenförmigen Antriebselement 11. Selbstverständlich kann das drehfest in der Injektionsvorrichtung 1 eingebaute Antriebselement 11 gemäß der vorliegenden Erfindung auch vor dem Führungselement 24 angeordnet sein, welche Anordnung beispielsweise aus der auf die Anmelderin zurückgehenden WO 87/02895 bekannt ist. Statt der in den Figuren dargestellten zylindrischen Ampulle 4 kann auch ein anderes Gefäß mit Kolben 5 als Produktbehälter verwendet werden.

Während vorstehend eine spezielle Ausführungsform, die zu der aus der EP 0 581 924 B1 der Anmelderin bekannten Injektionsvorrichtung ähnlich ist, beschrieben wurde, ist die vorliegende Erfindung grundsätzlich nicht auf diese spezielle Ausführungsform beschränkt. Vielmehr kann die vorliegende Erfindung grundsätzlich auf vergleichbare Injektionsvorrichtungen angewendet werden, wo zwischen drehfesten und drehbeweglichen Teilen der Vorrichtung ein Drehraster bzw. eine vergleichbare Verriegelungssperre vorgesehen ist. Ein Beispiel für eine solche weitere Ausführungsform ist aus der WO 97/17096 der Anmelderin bekannt. Diese Druckschrift sei ausdrücklich im Wege der Bezugnahme von der vorliegenden Anmeldung mit umfasst. Bei einer solchen der WO 97/17096 vergleichbaren Injektionsvorrichtung wird die Dosis durch Verdrehen eines Bedienknopfes eingestellt. Beim Verdrehen wird eine Gewindemutter entlang einem Gewinde verschoben, was den Abstand zwischen Gewindemutter und Kolben der Ampulle verändert. Bei axialer Betätigung des Bedienknopfes werden die Gewindestange und der Kolben um den so voreingestellten Abstand axial verstellt, um eine dosierte Produktausschüttung zu bewerkstelligen.

Bevorzugt wird die erfindungsgemäße Injektionsvorrichtung zur Selbsttherapie bei Diabetes-Patienten durch Selbstverabreichung von ärztlich verordneten Insulin-Dosen verwendet. Zu diesem Zweck weist die Injektionsvorrichtung bevorzugt eine Gauge-30- oder Gauge-31-Injektionsnadel 6 auf.

## Patentansprüche

1. Injektionsvorrichtung zur dosierten Verabreichung eines injizierbaren Produkts aus einem mit einem Kolben (5) versehenen Produktbehälter (4), insbesondere einer Ampulle, mit einer manuell antreibbaren röhrenartigen Betätigungseinrichtung (7),
a) die einen Bedienknopf (8), an dem axiale Bewegungen und Drehbewegungen ausführbar sind, ein drehfest mit dem Bedienknopf verbundenes Antriebselement (11), das Bewegungen des Bedienknopfes (8) folgt, und ein in Vorschubrichtung des Kolbens bewegbares Abtriebsglied (9, 19) umfasst, wobei
b) bei axialer Bewegung des Bedienknopfes (8) das Antriebselement (11) in der Vorschubrichtung des Kolbens (5) aus einer Ruhelage in eine Endlage und wieder zurück verschiebbar ist und diese Bewegung auf das mit dem Antriebselement (11) verbundene Abtriebsglied (9, 19) übertragbar ist, wobei
c) zwischen drehfesten und drehbaren Teilen der Vorrichtung ein Drehraster (20) vorgesehen ist, das zumindest zwei einander gegenüberliegende Rastelement (21, 23; 22, 23) mit einer Mehrzahl von zusammenwirkenden Rastvorsprüngen und Rastvertiefungen aufweist, wobei die Rastelemente (21-23) von einem ersten Rückstellmittel (16) in gegenseitige Anlage gebracht sind und beim Verdrehen des Bedienknopfes (8) in der Ruhelage die Rastvorsprünge an den Rastvertiefungen abgleiten, um ein Geräusch zu erzeugen, wobei
d) der Bedienknopf (8) nur in der Ruhelage drehbeweglich ist und
e) das Drehraster (20) ein erstes Rastelement (21) mit Rastvorsprüngen und Rastvertiefungen und ein dem ersten Rastelement (21) gegenüberliegendes zweites Rastelement (23) mit Rastvorsprüngen und Rastvertiefungen aufweist, die ein Verdrehen des Bedienknopfes (8) nur in eine erste Drehrichtung zulassen und das Verdrehen in die entgegengesetzte zweite Drehrichtung sperren,
**dadurch gekennzeichnet, dass**
f) das Drehraster (20) ein drittes Rastelement (22) mit Rastvorsprüngen und Rastvertiefungen aufweist, das dem zweiten Rastelement (23) gegenüberliegt, wobei das zweite Rastelement (23) und das dritte Rastelement (22) ein Verdrehen des Bedienknopfes (8) in die zweite Drehrichtung zulassen und in die erste Drehrichtung sperren, so dass der Bedienknopf (8) in der Ruhelage in beide Richtungen bewegbar ist.

2. Vorrichtung nach Anspruch 1, bei der das erste Rastelement (21) eine obere Scheibe, das dritte Rastelement (23) eine untere Scheibe und das zweite Rastelement (22) eine dazwischen angeordnete Zwischenscheibe sind.

3. Vorrichtung nach dem vorhergehenden Anspruch, bei der die Rastvorsprünge als in Umfangsrichtung der oberen bzw. unteren Scheibe (21, 22) bzw. der Zwischenscheibe (23) verlaufende sägezahnförmige Vorsprünge ausgebildet sind.

4. Vorrichtung nach einem der beiden vorhergehenden Ansprüche, bei der die Rastvorsprünge (41) auf einander gegenüberliegenden Seiten der Zwischenscheibe (23) an in Vorschubrichtung des Kolbens (5) betrachtet zueinander fluchtenden Winkelpositionen angeordnet sind.

5. Vorrichtung nach Anspruch 2 oder 3, bei der die Rastvorsprünge (41) auf der oberen und unteren Scheibe (21, 22) in der Ruhestellung des Bedienknopfes (8) an in Vorschubrichtung des Kolbens (5) betrachtet relativ zueinander versetzten Winkelpositionen angeordnet sind.

6. Vorrichtung nach einem der vorhergehenden Ansprüche, bei der der Bedienknopf (8) nur bei eingerastetem Drehraster (20) axial in die Endlage verstellbar ist.

7. Vorrichtung nach Anspruch 6, bei der am Außenumfang des Bedienknopfes (8) oder an einem zu diesem drehfest angeordneten Element eine Anzahl von Rastkörpern (14) angeordnet sind, deren Anzahl der Anzahl von Rastvorsprüngen auf Stirnseiten der Rastelemente (21-23) des Drehrasters (20) entspricht.

8. Vorrichtung nach einem der vorhergehenden Ansprüche, bei der ein Ampullenhalter (29), der die untere Scheibe (22; 36) ausbildet oder mit dieser gekoppelt ist, gegen die Rückstellkraft eines zweiten Rückstellelements (35) axial in Richtung des Bedienknopfs (8) zurückgeschoben ist, so dass das Drehraster (20) einrastet.

9. Vorrichtung nach Anspruch 8, bei der das zweite Rückstellmittel (35) zwischen einem hinteren Teil (3) und dem Ampullenhalter (29) sitzt.

10. Vorrichtung nach Anspruch 8 oder 9, bei der eine Länge der Ampulle (4) und eine Länge des vorderen Teils (2) so aufeinander abgestimmt sind, dass das Drehraster (20) einrastet, wenn die Ampulle (4) in das vordere Teil (2) eingesetzt ist und das vordere und hintere Teil (2, 3) miteinander verbunden sind.

11. Vorrichtung nach Anspruch 10, bei der das zweite Rückstellmittel (35) eine Druckfeder ist, die nicht vollständig komprimiert ist, wenn das vordere und hintere Teil (2, 3) miteinander verbunden sind.

12. Vorrichtung nach einem der Ansprüche 8 bis 11, bei der die Rückstellkraft des zweiten Rückstellmittels (35) kleiner ist als die Rückstellkraft des ersten Rückstellmittels (16).

13. Vorrichtung nach einem der vorhergehenden Ansprüche, bei der die Betätigungseinrichtung (7) ferner ein Führungselement (24) für das Abtriebselement umfasst.

14. Vorrichtung nach Anspruch 13, bei der das Führungselement (24) fest mit einem hinteren Teil (3) der Vorrichtung (1) verbunden ist und das Abtriebsglied (9, 19) im Führungselement (24) drehfest gelagert ist.

15. Vorrichtung nach Anspruch 14, bei der das ortsfeste Führungselement (24) vor dem Antriebselement (11) angeordnet ist.

16. Vorrichtung nach Anspruch 14, bei der das ortsfeste Führungselement (24) hinter dem Antriebselement (11) angeordnet ist.

17. Vorrichtung nach einem der vorhergehenden Ansprüche, bei der das in Vorschubrichtung des Kolbens bewegbare Abtriebsglied (9, 19) drehfest in Bezug auf ein Gehäuse der Vorrichtung ist und bei der in der Ruhelage durch eine Drehbewegung des Bedienknopfes (8) das vom Kolben (5) beabstandete Abtriebsglied (9, 19) durch das Antriebselement (11) in der Vorschubrichtung entsprechend einem für die jeweils zu injizierende Produktmenge erforderlichen Kolbenweg bewegbar ist, wobei das Abtriebsglied (9, 19) vom Kolben (5) beabstandet bleibt und während des Überganges des Antriebselements (11) von der Ruhelage zur Endlage das Abtriebsglied (9, 19) am Kolben (5) zum Anliegen kommt, um den Kolben (5) um den so vorgewählten Kolbenweg zu verschieben.

## Claims

1. An injection device for the metered administration of an injectable product from a product container (4) provided with a plunger (5), in particular an ampoule, having a manually drivable tubular actuating device (7)
a) which includes an operating knob (8) at which axial movements and rotary movements can be effected, a drive element (11) which is non-rotatably connected to the operating knob and which follows movements of the operating knob (8), and a driven member (9, 19) movable in the advance direction of the plunger, wherein
b) upon axial movement of the operating knob (8) the drive element (11) is displaceable in the advance direction of the plunger (5) out of a rest position into an end position and back again and said movement can be transmitted to the driven member (9, 19) which is connected to the drive element (11), wherein
c) provided between non-rotatable and rotatable parts of the device is a rotary latching means (20) having at least two mutually oppositely disposed latching elements (21, 23; 22, 23) with a plurality of co-operating latching projections and latching recesses, wherein the latching elements (21-23) are brought into mutual contact by a first return means (16) and upon rotation of the operating knob (8) in the rest position the latching projections slide against the latching recesses in order to produce a sound, wherein
d) the operating knob (8) is rotationally movable only in the rest position and
e) the rotary latching means (20) has a first latching element (21) with latching projections and latching recesses and a second latching element (23) which is opposite to the first latching element (21) and has latching projections and latching recesses which allow a rotary movement of the operating knob (8) only in a first rotary direction and which block the rotary movement in the opposite second rotary direction,
**characterised in that**
f) the rotary latching means (20) has a third latching element (22) with latching projections and latching recesses, which is disposed opposite to the second latching element (23), wherein the second latching element (23) and the third latching element (22) allow a rotary movement of the operating knob (8) in the second rotary direction and to block same in the first rotary direction so that the operating knob (8) in the rest position is movable in both directions.

2. A device according to claim 1 wherein the first latching element (21) is an upper disc, the third latching element (23) is a lower disc and the second latching element (22) is an intermediate disc arranged therebetween.

3. A device according to the preceding claim wherein the latching projections are in the form of sawtooth-shaped projections extending in the peripheral direction of the upper and lower discs (21, 22) and the intermediate disc (23) respectively.

4. A device according to one of the preceding claims wherein the latching projections (41) are arranged on mutually opposite sides of the intermediate disc (23) at angular positions which are in alignment with each other when considered in the advance direction of the plunger (5).

5. A device according to claim 2 or claim 3 wherein the latching projections (41) on the upper and lower discs (21, 22) in the rest position of the operating knob (8) are arranged at angular positions which are displaced relative to each other when considered in the advance direction of the plunger (5).

6. A device according to one of the preceding claims wherein the operating knob (8) is axially displaceable into the end position only when the rotary latching means (20) is in latching engagement.

7. A device according to claim 6 wherein arranged at the outer periphery of the operating knob (8) or at an element arranged non-rotatably in relation therewith is a number of latching bodies (14), the number of which corresponds to the number of latching projections on end faces of the latching elements (21-23) of the rotary latching means (20).

8. A device according to one of the preceding claims wherein an ampoule holder (29) which forms the lower disc (22; 36) or is coupled thereto is pushed back axially against the return force of a second return element (35) in the direction of the operating knob (8) so that the rotary latching means (20) comes into latching engagement.

9. A device according to claim 8 wherein the second return means (35) is disposed between a rear portion (3) and the ampoule holder (29).

10. A device according to claim 8 or claim 9 wherein a length of the ampoule (4) and a length of the front portion (2) are so matched to each other that the rotary latching means (20) comes into latching engagement when the ampoule (4) is fitted into the front portion (2) and the front and rear portions (2, 3) are connected together.

11. A device according to claim 10 wherein the second return means (35) is a compression spring which is not completely compressed when the front and rear portions (2, 3) are connected together.

12. A device according to one of claims 8 to 11 wherein the return force of the second return means (35) is less than the return force of the first return means (16).

13. A device according to one of the preceding claims wherein the actuating device (7) further includes a guide element (24) for the driven element.

14. A device according to claim 13 wherein the guide element (24) is fixedly connected to a rear portion (3) of the device (1) and the driven member (9, 19) is mounted non-rotatably in the guide element (24).

15. A device according to claim 14 wherein the stationary guide element (24) is arranged in front of the drive element (11).

16. A device according to claim 14 wherein the stationary guide element (24) is arranged behind the drive element (11).

17. A device according to one of the preceding claims wherein the driven member (9, 19) which is movable in the advance direction of the plunger is non-rotatable with respect to a casing of the device and wherein in the rest position the driven member (9, 19) which is spaced from the plunger (5) is movable by a rotary movement of the operating knob (8) by the drive element (11) in the advance direction in accordance with a plunger travel required for the respective amount of product to be injected, wherein the driven member (9, 19) remains spaced from the plunger (5) and during the transition of the drive element (11) from the rest position to the end position the driven member (9, 19) comes to bear against the plunger (5) in order to displace the plunger (5) by the plunger travel which is preselected in that way.

## Revendications

1. Dispositif d'injection pour l'administration dosée d'un produit injectable à partir d'un récipient à produit (4) pourvu d'un piston (5), en particulier d'une ampoule, comportant un moyen d'actionnement (7) de forme tubulaire susceptible d'être entraîné manuellement,
a) qui comprend un bouton de manipulation (8) au niveau duquel peuvent être exécutés des mouvements axiaux et des mouvements de rotation, un élément menant (11) relié solidairement en rotation au bouton de manipulation, qui suit des mouvements du bouton de manipulation (8), et un organe mené (9, 19) mobile en direction d'avance du piston, dans lequel
b) lors d'un mouvement axial du bouton de manipulation (8), l'élément menant (11) est mobile en translation à partir d'une position de repos jusque dans une position terminale et en retour, et ce mouvement est transmissible à l'organe mené (9, 19) relié à l'organe menant (11), dans lequel
c) entre des parties solidaires en rotation et des parties mobiles en rotation du dispositif, il est prévu une trame tournante (20) qui comprend au moins deux éléments d'enclenchement (21, 23 ; 22, 23) opposés l'un à l'autre munis d'une multitude de saillies d'enclenchement et de renfoncements d'enclenchement en coopération, les éléments d'enclenchement (21 à 23) étant amenés en appui mutuel par un premier moyen de rappel (16) et lors d'une rotation du bouton de manipulation (8), dans la position de repos, les saillies d'enclenchement glissent le long des renfoncements d'enclenchement pour produire un bruit, dans lequel
d) le bouton de manipulation (8) est mobile en rotation uniquement dans la position de repos, et
e) la trame tournante (20) comprend un premier élément d'enclenchement (21) avec des saillies d'enclenchement et des renfoncements d'enclenchement, et un second élément d'enclenchement (23) opposé au premier élément d'enclenchement (21) et muni de saillies d'enclenchement et de renfoncements d'enclenchement qui permettent une rotation du bouton de manipulation (8) uniquement dans une première direction de rotation et qui bloquent la rotation dans la seconde direction de rotation opposée,
**caractérisé en ce que**
f) la trame tournante (20) comprend un troisième élément d'enclenchement (22) muni de saillies d'enclenchement et de renfoncements d'enclenchement et opposé au second élément d'enclenchement (23), le second élément d'enclenchement (23) et le troisième élément d'enclenchement (22) permettant une rotation du bouton de manipulation (8) dans la seconde direction de rotation et le bloquant dans la première direction de rotation, de sorte que dans la position de repos, le bouton de manipulation (8) est mobile dans les deux directions.

2. Dispositif selon la revendication 1, dans lequel le premier élément d'enclenchement (21) est une plaque supérieure, le troisième élément d'enclenchement (23) est une plaque inférieure et le second élément d'enclenchement (22) est une plaque intermédiaire agencée entre les deux.

3. Dispositif selon la revendication précédente, dans lequel les saillies d'enclenchement sont réalisées sous forme de saillies en forme de dents de scie s'étendant en direction périphérique de la plaque supérieure ou inférieure (21, 22) ou de la plaque intermédiaire (23).

4. Dispositif selon l'une des deux revendications précédentes, dans lequel les saillies d'enclenchement (41) sont agencées sur des côtés mutuellement opposés de la plaque intermédiaire (23) dans des positions angulaires alignées l'une vers l'autre, vues en direction d'avance du piston (5).

5. Dispositif selon la revendication 2 ou 3, dans lequel, dans la position de repos du bouton de manipulation (8), les saillies d'enclenchement (41) sur les plaques supérieure et inférieure (21, 22) sont agencées dans des positions angulaires décalées l'une de l'autre, vues en direction d'avance du piston (5).

6. Dispositif selon l'une des revendications précédentes, dans lequel le bouton de manipulation (8) est réglable axialement dans la position terminale uniquement lorsque la trame tournante (20) est enclenchée.

7. Dispositif selon la revendication 6, dans lequel un certain nombre de corps d'enclenchement (14) sont agencés sur la périphérie extérieure du bouton de manipulation (8) ou sur un élément agencé solidairement en rotation par rapport à celui-ci, dont le nombre correspond au nombre de saillies d'enclenchement sur des côtés frontaux des éléments d'enclenchement (21 à 23) de la trame tournante (20).

8. Dispositif selon l'une des revendications précédentes, dans lequel un porte-ampoule (29) qui constitue la plaque inférieure (22 ; 36) ou qui est couplé à celle-ci est repoussé axialement en direction du bouton de manipulation (8) à l'encontre de la force de rappel d'un second élément de rappel (35), de sorte que la trame tournante (20) vient s'enclencher.

9. Dispositif selon la revendication 8, dans lequel le second moyen de rappel (35) est logé entre une partie arrière (3) et le porte-ampoule (29).

10. Dispositif selon la revendication 8 ou 9, dans lequel une longueur de l'ampoule (4) et une longueur de la partie avant (2) sont adaptées l'une à l'autre de telle sorte que la trame tournante (20) vient s'enclencher lorsque l'ampoule (4) est mise en place dans la partie avant (2) et que les parties avant et arrière (2, 3) sont reliées l'une à l'autre.

11. Dispositif selon la revendication 10, dans lequel le second moyen de rappel (35) est un ressort de compression qui n'est pas complètement comprimé lorsque les parties avant et arrière (2, 3) sont reliées l'une à l'autre.

12. Dispositif selon l'une des revendications 8 à 11, dans lequel la force de rappel du second moyen de rappel (35) est inférieure à la force de rappel du premier moyen de rappel (16).

13. Dispositif selon l'une des revendications précédentes, dans lequel le dispositif d'actionnement (7) comprend en outre un élément de guidage (24) pour l'élément mené.

14. Dispositif selon la revendication 13, dans lequel l'élément de guidage (24) est fermement relié à une partie arrière (3) du dispositif (12), et l'organe mené (9, 19) est monté solidairement en rotation dans l'élément de guidage (24).

15. Dispositif selon la revendication 14, dans lequel l'élément de guidage stationnaire (24) est agencé en avant de l'élément menant (11).

16. Dispositif selon la revendication 14, dans lequel l'élément de guidage stationnaire (24) est agencé en arrière de l'élément menant (11).

17. Dispositif selon l'une des revendications précédentes, dans lequel l'organe mené (9, 19) mobile en direction d'avance du piston est solidaire en rotation par rapport à un boîtier du dispositif, et dans lequel, dans la position de repos, l'organe mené (9, 19) espacé du piston (5) est mobile par un mouvement de rotation du bouton de manipulation (8), au moyen de l'élément menant (11) dans la direction d'avance en correspondance d'une course de piston nécessaire pour la quantité de produit respective à injecter, l'organe mené (9, 19) restant espacé du piston (5), et pendant la transition de l'élément menant (11) depuis la position de repos vers la position terminale, l'organe mené (9, 19) vient en appui contre le piston (5) pour déplacer le piston (5) de la course de piston ainsi présélectionnée.
